# EUROPEAN PATENT SPECIFICATION

(11) **EP 1 922 088 B1**
(45) Date of publication and mention of the grant of the patent: **10.07.2013**
(21) Application number: 06789385.9
(22) Date of filing: 03.08.2006
(51) Int. Cl.: A61L 2/20, A47L 15/24, A47L 15/00

(54) **CONVEYOR DISHWASHER**
FÖRDER-GESCHIRRSPÜLER
LAVE-VAISSELLE À CONVOYEUR

(30) Priority: 18.08.2005 DE 102005039139
(43) Date of publication of application: 21.05.2008
(73) Proprietor: Premark FEG L.L.C., Wilmington, Delaware 19801 (US)
(72) Inventor: BERNER, Dietrich, 73550 Waldstetten (DE); PADTBERG, Klaus, 77652 Offenburg (DE)
(74) Representative: Meissner, Bolte & Partner
(86) International application number: PCT/US2006/030408
(87) International publication number: WO 2007/021577

(56) References cited:
- EP-A- 1 042 983
- WO-A-03/037801
- WO-A-2004/100755
- WO-A-2005/063109
- DE-A1- 19 644 438
- US-A1- 2002 153 021

## Description

The invention concerns a conveyor ware washer, particularly a commercial conveyor ware washer of the type commonly configured in the form of a flight-type ware washer or in the form of a rack conveyor ware washer.

Conveyor dishwashers are available in the form of a belt conveyor dishwasher (flight-type dishwasher, flight-type ware washer) and in the form of a rack conveyor dishwasher or rack conveyor ware washer.

Conveyor dishwashers are known from the following publications, for example: United States Patents 3,789,860; 4,231,806; German Patents DE 196 44 438 C2; DE 198 29 560 C2.

In various applications or branches of the industry and in various countries, the reduction in microbial contamination achieved by dishwashers is not sufficient to meet the standards specified, e.g., according to DIN 10510. This requires additional disinfectant measures, usually thermal disinfection.

It would be desirable to provide a conveyor dishwasher that provides a good disinfectant effect without consuming as much electric power as required for thermal disinfection in the past. This object is achieved by a conveyor dishwasher having the features of Claim 1.

Accordingly, the invention relates to a conveyor dishwasher which is characterized in that an ozone feed mechanism is provided for adding ozone from an ozone source into the final rinse liquid upstream from the final rinse nozzles provided for spraying final rinse liquid onto the wash ware.

The present invention achieves a very good and adequate disinfection effect without requiring thermal disinfection, so the approach according to the present invention also saves on natural resources, in particular electric power.

If the dishwasher contains a water heater, e.g., a boiler, for heating the final rinse liquid, its temperature is preferably lowered in comparison with the state of the art. Therefore ozone can be dissolved better in the final rinse liquid. Ozone has a disinfecting effect. Any microorganisms that might still remain on the wash ware after washing are killed with the help of the ozonized final rinse liquid. Such microorganisms include in particular algae, fungi, yeasts, bacteria and viruses. The ozone may be in any suitable form, e.g., even in a solid, liquid or gaseous carrier, e.g., in an ozone-air mixture.

Final rinse liquid may be fresh water with or without a rinse aid. The wash liquid is typically water to which detergent is added.

Useful background art and embodiments of the invention are described in greater detail below with reference to the accompanying drawings on the basis of examples. The drawings show:
Figure 1 a schematic side view of a conveyor dishwasher not forming part of the invention but representing background art that is useful for understanding the invention,
Figure 2 a schematic detail of the dishwasher from Figure 1 illustrating another not forming part of the invention but representing background art that is useful for understanding the invention,
Figure 3 a schematic detail of the dishwasher from Figure 1 illustrating another not forming part of the invention but representing background art that is useful for understanding the invention,
Figure 4 a schematic detail of the dishwasher from Figure 1 illustrating one embodiment of the invention,
Figure 5 a schematic detail of the dishwasher from Figure 1 illustrating another embodiment of the invention,
Figure 6 a schematic detail of the dishwasher from Figure 1 illustrating another embodiment of the invention.

By way of example, the conveyor ware washer according to this invention may be a flight-type ware washer in which the wash ware to be cleaned is conveyed by a conveyor belt through a machine or a rack-type conveyor ware washer in which the wash ware is conveyed in racks on a conveyor device through the machine:

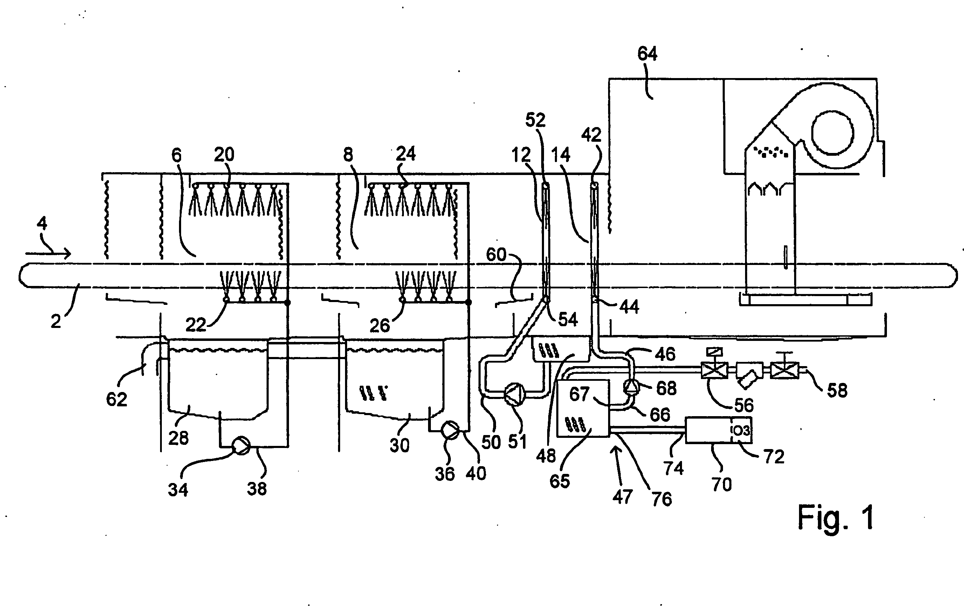

The conveyor dishwasher in Figure 1 contains a conveyor device 2, the direction of conveyance 4 of which is indicated by an arrow. The wash ware (not shown) may be of any type, e.g., plates, cups, glasses, utensils, trays, pots and pans.

The conveyor dishwasher contains at least one wash zone, e.g., according to the drawings, two (or more) wash zones 6 and 8 arranged in succession in the direction of conveyance 4 for washing the wash ware with wash liquid, followed by a post-wash zone 12 for post-washing of the wash ware using post-wash liquid and following that, at least one final rinse zone for the final rinse of the wash ware with final rinse liquid.

The wash liquid is water to which detergent is added by metered addition. The final rinse liquid may be fresh water with or without added rinse aid.

In each wash zone 6 and 8 there are upper and lower wash nozzles 20, 22 and 24, 26 for spraying wash liquid onto the wash ware in the conveyor device 2. The wash zones 6 and 8 are each equipped with a wash tank 28 and 30, respectively, in which the wash liquid sprayed by the wash nozzles 20, 22 and 24, 26 collects. Wash liquid is recirculated back from the wash tanks 28 and 30 to the wash nozzles 20, 22 and 24, 26, respectively, through a wash liquid feed line 38 or 40, respectively, by means of a pump 34 or 36. The wash liquid feed line 38 or 40 and the respective wash tank 28 or 30, respectively, each forms a wash liquid recirculation line 28/38 and 30/40. Each wash liquid recirculation line 28/38 and 30/40 together with the respective wash zone 6 or 8 and the respective wash nozzles 20, 22 or 24, 26 forms a wash liquid recirculation system.

The final rinse zone is equipped with upper and lower final rinse nozzles 42 and 44 to which final rinse liquid can be supplied through a final rinse liquid feed line 46 of a final rinse liquid feed mechanism 47. The final rinse liquid sprayed by the final rinse nozzles 42 and 44 collects in a post-wash tank 48 in the wash zone 12. Post-wash liquid is supplied from the post-wash tank 48 by means of a pump 51 through a post-wash liquid supply line 50 to the upper and lower post-wash nozzles 52 and 54 of the post-wash zone 12. Some of the post-wash liquid sprayed by the post-wash nozzles 52 and 54 goes into the post-wash tank 48 and some of it goes through a guide element 60 into the last wash tank 30. The post-wash liquid is thus preused final rinse liquid.

For this reason, the post-wash zone may also be referred to as a pre-rinse zone, the post-wash liquid may be referred to as pre-rinse liquid; the post-wash tank 48 may be referred to as the pre-rinse tank and the post-wash nozzle 52 and 54 may be referred to as pre-rinse nozzles.

The wash tanks 28 and 30 and the post-wash zone 48 form a liquid cascade system in which the liquid overflows from one tank to the other in a direction opposite the direction of conveyance 4 (the "transport direction") of the conveyance device 2. Thus liquid flows from the post-wash tank 48 into the wash tank 30 that is the last in the direction of conveyance and from there into the preceding wash tank 28. The last wash tank 28 in the direction of flow, which is also the first wash tank in the direction of conveyance 4, is preferably provided with a drain 62 for dirty liquid.

A dry zone 64 for drying the cleaned wash ware may be provided in the direction of conveyance 4 downstream from the final rinse zone 14.

The invention also relates to conveyor dishwashers having a post-wash zone 12. In this case, the liquid sprayed by the final rinse nozzles 42 and 44 in the final rinse zone 14 is collected by a guide element, e.g., a bottom or a bottom pan or a tank of the final rinse zone 14 and conveyed into the wash tank 30 which is the last in the direction of conveyance 4.

The final rinse liquid feed mechanism 47 may have a fresh water connection 56 for connecting to a fresh water feed line 58. Furthermore, a water heater 65, preferably a boiler, may be provided for heating the final rinse liquid if necessary with respect to the wash ware to be cleaned.

If the water pressure of the fresh water feed line 58 is high enough and constant enough, the final rinse liquid feed line 46 of the final rinse nozzles 42 and 44 may be connected to an outlet 67 of the water heater 65 without an intermediate pump in between. If the fresh water pressure is not enough to supply final rinse liquid to the final rinse liquid spray nozzles 42 and 44, a pump 68 may be provided between the fresh water feed line 58 and the final rinse liquid feed lined 46. The pump 68 may be arranged between the outlet 67 of the water heater 65 and the final rinse liquid feed line 46 either directly or via a connecting line 66.

If desired, rinse aid may be added to the fresh water of the fresh water feed line 58 at any suitable location upstream from the final rinse nozzles 42, 44.

An ozone-adding mechanism 70 is provided for adding ozone 03 or an ozone gas mixture from an ozone source 72 to the final rinse liquid upstream from the final rinse nozzles 42 and 44. The ozone source 72 is an ozone gas source from which ozone or preferably an ozone gas mixture is supplied through the ozone addition mechanism 70 to the final rinse liquid before the latter is sprayed through the final rinse nozzles 42, 44. The ozone gas mixture is preferably an ozone-air mixture.

Figure 1 shows an ozone dispensing line 74 of the ozone-adding mechanism 70 which is connected to the water heater 65.

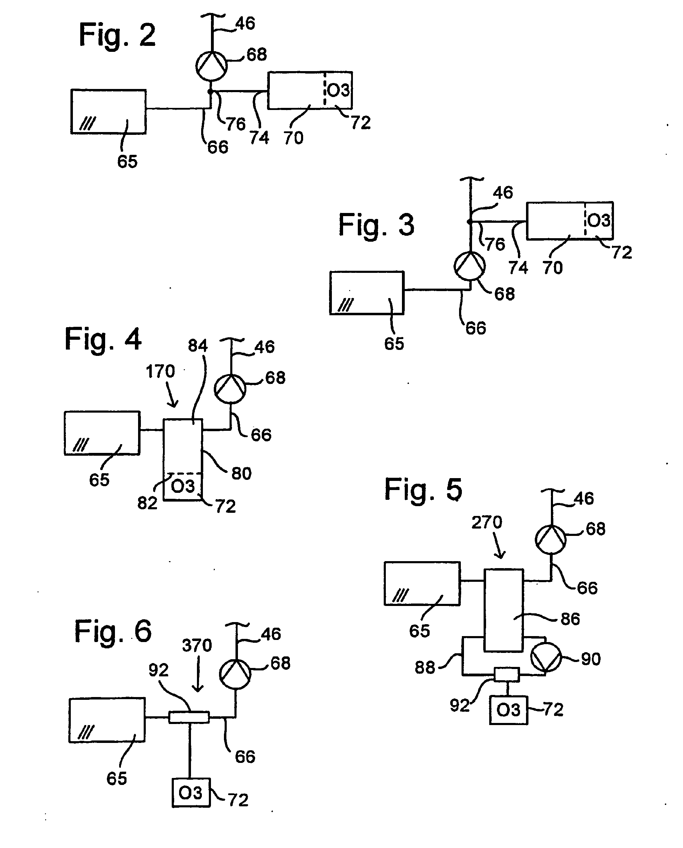

In another example according to Figure 2, the ozone-dispensing end 76 of the ozone-dispensing line 74 may be arranged on the connecting line 66 between the water heater 65 and the final rinse pump 68. In another example according to Figure 3, the ozone-dispensing end 76 of the ozone-dispensing line 74 is arranged on the final rinse liquid feed line 46 between its pump 68 and the final rinse nozzles 42 and 44.

Figures 4, 5 and 6 illustrate various possibilities for adding ozone to the final rinse liquid according to the invention.

In the embodiment 170 of an ozone feed mechanism shown in Figure 4, a supply tank 80 is provided in the connecting line 66, for example, in which ozone 03 can penetrate from an ozone source 72 through a gas-permeable membrane 82 into the final rinse liquid which flows on the other side of the membrane through a final rinse liquid chamber 84.

In the embodiment 270 of an ozone-adding mechanism as illustrated in Figure 5, a liquid distributor 86 is provided; the final rinse liquid flows from the water heater 65 to the final rinse liquid supply line 46 through this liquid distributor, where a portion of the final rinse liquid flows to and from a recirculation system 88 and is recirculated there. A pump 90 and a Venturi element or an injector element 92 which draws in ozone 03 from an ozone source 72 into the recirculated final rinse liquid are provided in the recirculation system. The Venturi element or injector element 92 creates a vacuum according to the Venturi principle or the injector principle. The portion of the final rinse liquid enriched with ozone mixes in the liquid distributor 86 with the portion that has not yet been enriched with ozone before the two portions are then mixed together and flow to the final rinse nozzles 42 and 44.

In the embodiment 370 in Figure 6, a Venturi element or an injector element 92 is arranged directly in the final rinse liquid supply path to the final rinse nozzles 42 and 44 to draw in ozone 03 from an ozone source 72. The Venturi element or injector element 92 may be arranged upstream or downstream from the final rinse pump 68, e.g., on the final rinse liquid supply line 46 or, as shown in Figure 6, on the connecting line 66 from the heat exchanger 65 to the final rinse pump 68.

## Claims

1. A conveyor dishwasher, comprising:
at least one wash zone (6, 8) having wash nozzles (20, 22, 24, 26) for spraying wash liquid and a wash liquid recirculation line (28/38, 30/40) including a wash tank (28, 30) for collecting at least some sprayed wash liquid and a wash liquid supply line (38, 40) with a pump (34, 36) for supplying wash liquid from the wash tank (28, 30) to the wash nozzles (20, 22, 24, 26);
a final rinse zone (14) having final rinse nozzles (42, 44) for spraying final rinse liquid;
a conveyor device (2) for conveying wares through the wash zone and the final rinse zone, whereby a transport direction (4) of the conveyor device (2) is directed from the wash zone to the final rinse zone;
**characterized in that**
an ozone-adding mechanism (70, 170, 270, 370) for adding ozone (03) from an ozone source (72) to final rinse liquid is provided upstream from the final rinse nozzles (42, 44);
wherein the ozone- adding mechanism (170) includes an ozone chamber (72) and a final rinse liquid chamber (84) through which final rinse liquid flows, said final rinse liquid chamber and ozone chamber being separated from one another by a gas- permeable membrane (82) through which ozone or an ozone gas mixture is dispensed from the ozone chamber (72) into the final rinse liquid chamber; or
wherein the ozone-adding mechanism (270) has a Venturi element or an injector element (92) which has final rinse liquid flowing through it and thereby draws in ozone or an ozone gas mixture into the final rinse liquid.

2. A conveyor dishwasher according to claim 1, wherein the Venturi element or injector element (92) is arranged in a recirculation system (88) which has a pump (90) and a liquid distributor (86), a portion of the final rinse liquid being branched off in the liquid distributor (86) into the recirculation system (88) and, after ozonization, being returned back to the remainder of the final rinse liquid.

3. A conveyor dishwasher according to claim 1, wherein the Venturi element or injector element (92) is arranged in a line segment (46, 66) through which the final rinse liquid flows to the final rinse nozzles (42, 44) so that in this element (92) the final rinse liquid draws in ozone or an ozone gas mixture from an ozone source (72).

## Patentansprüche

1. Fördergeschirrspüler, aufweisend:
mindestens einen Waschbereich (6, 8) mit Waschdüsen (20, 22, 24, 26) zum Sprühen von Waschflüssigkeit und einer Waschflüssigkeitwiederumlaufleitung (28/38, 30/40), die einen Waschtank (28, 30) zum Auffangen von mindestens einiger der gesprühten Waschflüssigkeit und eine Waschflüssigkeitszufuhrleitung (38, 40) mit einer Pumpe (34, 36) zum Zuführen von Waschflüssigkeit von dem Waschtank (28, 30) zu den Waschdüsen (20, 22, 24, 26) enthält;
einen Endspülbereich (14) mit Endspüldüsen (42, 44) zum Sprühen von Endspülflüssigkeit;
eine Fördervorrichtung (2) zum Befördern von Artikeln durch den Waschbereich und den Endspülbereich, wobei eine Transportrichtung (4) der Fördervorrichtung (2) vom Waschbereich zum Endspülbereich gerichtet ist;
**dadurch gekennzeichnet, dass**
ein Ozonzusatzmechanismus (70, 170, 270, 370) zum Zusetzen von Ozon (03) aus einer Ozonquelle (72) zu der Endspülflüssigkeit den Endspüldüsen (42, 44) vorgeschaltet vorgesehen ist;
wobei der Ozonzusatzmechanismus (170) eine Ozonkammer (72) und eine Endspülflüssigkeitskammer (84), durch die Endspülflüssigkeit strömt, enthält, wobei die Endspülflüssigkeitskammer und die Ozonkammer durch eine gasdurchlässige Membran (82) voneinander getrennt sind, durch die Ozon oder ein Ozongasgemisch aus der Ozonkammer (72) in die Endspülflüssigkeitskammer abgegeben wird; oder
wobei der Ozonzusatzmechanismus (270) ein Venturi-Element oder ein Einspritzelement (92) aufweist, durch das Endspülflüssigkeit strömt und das dadurch Ozon oder ein Ozongasgemisch in die Endspülflüssigkeit einzieht.

2. Fördergeschirrspüler nach Anspruch 1, wobei das Venturi-Element oder Einspritzelement (92) in einem Wiederumlaufsystem (88) angeordnet ist, das eine Pumpe (90) und einen Flüssigkeitsverteiler (86) aufweist, wobei ein Anteil der Endspülflüssigkeit in den Flüssigkeitsverteiler (86) in das Wiederumlaufsystem (88) abgeleitet und nach der Ozonisierung zum Rest der Endspülflüssigkeit rückgeführt wird.

3. Fördergeschirrspüler nach Anspruch 1, wobei das Venturi-Element oder Einspritzelement (92) in einem Leitungssegment (46, 66) angeordnet ist, durch das die Endspülflüssigkeit zu den Endspüldüsen (42, 44) strömt, sodass die Endspülflüssigkeit in diesem Element (92) Ozon oder ein Ozongasgemisch aus einer Ozonquelle (72) einzieht.

## Revendications

1. Lave-vaisselle à convoyeur, comprenant :
au moins une zone de lavage (6, 8), ayant des buses de lavage (20, 22, 24, 26) pour pulvériser du liquide de lavage et une ligne de recirculation de liquide de lavage (28/38, 30/40) comportant un réservoir de lavage (28, 30) pour recueillir au moins une partie du liquide de lavage pulvérisé et une ligne d'alimentation en liquide de lavage (38, 40) avec une pompe (34, 36) pour fournir du liquide de lavage depuis le réservoir de lavage (28, 30) aux buses de lavage (20, 22, 24, 26) ;
une zone de rinçage final (14) ayant des buses de rinçage final (42, 44) pour pulvériser du liquide de rinçage final ;
un dispositif convoyeur (2) pour transporter de la vaisselle à travers la zone de lavage et la zone de rinçage final, une direction de transport (4) du dispositif convoyeur (2) étant orientée depuis la zone de lavage vers la zone de rinçage final ; **caractérisé en ce**
**qu'**un mécanisme d'ajout d'ozone (70, 170, 270, 370) pour ajouter de l'ozone (03) provenant d'une source d'ozone (72) au liquide de rinçage final est prévu en amont des buses de rinçage final (42, 44) ;
le mécanisme d'ajout d'ozone (170) comportant une chambre d'ozone (72) et une chambre de liquide de rinçage final (84) à travers laquelle s'écoule du liquide de rinçage final, ladite chambre de liquide de rinçage final et ladite chambre d'ozone étant séparées l'une de l'autre par une membrane perméable aux gaz (82) à travers laquelle de l'ozone ou un mélange d'ozone gazeux est distribué depuis la chambre d'ozone (72) jusque dans la chambre de liquide de rinçage final ; ou
le mécanisme d'ajout d'ozone (270) ayant un élément venturi ou un élément d'injecteur (92) à travers lequel s'écoule du liquide de rinçage final et qui introduit ainsi de l'ozone ou un mélange d'ozone gazeux dans le liquide de rinçage final.

2. Lave-vaisselle à convoyeur selon la revendication 1, dans lequel l'élément venturi ou l'élément d'injecteur (92) est prévu dans un système de recirculation (88) qui présente une pompe (90) et un distributeur de liquide (86), une portion du liquide de rinçage final étant déviée dans le distributeur de liquide (86) jusque dans le système de recirculation (88), et, après l'ozonisation, étant ramenée au reste du liquide de rinçage final.

3. Lave-vaisselle à convoyeur selon la revendication 1, dans lequel l'élément venturi ou l'élément d'injecteur (92) est prévu dans un segment de ligne (46, 66) à travers lequel le liquide de rinçage final s'écoule jusqu'aux buses de rinçage final (42, 44) de telle sorte que dans cet élément (92), le liquide de rinçage final aspire de l'ozone ou un mélange d'ozone gazeux provenant d'une source d'ozone (72).
